(19) Europäisches Patentamt
European Patent Office
Office européen des brevets

(11) **EP 2 717 872 B1**

(12) **EUROPEAN PATENT SPECIFICATION**

(45) Date of publication and mention
of the grant of the patent:
**06.04.2016 Bulletin 2016/14**

(21) Application number: **12735335.7**

(22) Date of filing: **05.06.2012**

(51) Int Cl.:
*A61K 31/4188* (2006.01)  *A61K 33/24* (2006.01)
*A61K 36/258* (2006.01)  *A61K 36/25* (2006.01)
*A61P 3/10* (2006.01)

(86) International application number:
**PCT/IB2012/052815**

(87) International publication number:
**WO 2012/168854 (13.12.2012 Gazette 2012/50)**

(54) **SYNERGISTIC COMBINATION FOR THE TREATMENT OF TYPE 2 DIABETES MELLITUS**

SYNERGISTISCHE KOMBINATION ZUR BEHANDLUNG VON DIABETES TYP 2

COMBINAISON SYNERGIQUE DESTINÉE AU TRAITEMENT DU DIABÈTE SUCRÉ DE TYPE 2

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.06.2011 ES 201130956**

(43) Date of publication of application:
**16.04.2014 Bulletin 2014/16**

(73) Proprietor: **PRI, S. A.**
**463 Clémency (LU)**

(72) Inventor: **RADWAN, Farouk**
**Laval, Québec H7T-2Y4 (CA)**

(74) Representative: **Gallego Jiménez, José Fernando
Ingenias Creaciones, Signos e Invenciones
S.L.P.
Avda. Diagonal 421, 2
08008 Barcelona (ES)**

(56) References cited:
**WO-A1-02/09693**      **CN-A- 101 390 902**
**US-A1- 2008 102 137**

Remarks:
The file contains technical information submitted after
the application was filed and not included in this
specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Technical field

The present invention relates to a new combination of mineral ingredients, vitamins and herbal products effective for the treatment of type 2 diabetes mellitus.

### Background art

Diabetes mellitus is one of the most frequent chronic diseases in the world and its incidence shows a clear upward trend. Thus, for example, according to a recently published study (Shaw et al., Global estimates of the prevalence of diabetes for 2010 and 2030, Diabetes Res.Clin.Pract., 2010, 87, 4-14), it is estimated that the prevalence of this disease will be of 6.4% in 2010, with about 285 million people affected worldwide, and with a forecast of 439 million affected in 2030.

The term diabetes mellitus encompasses a group of diseases characterised for provoking hyperglycaemia, i.e., a blood glucose level higher than normal. Diabetes is a serious pathology as this high blood glucose level can cause multiple damages in the organism, for example at blood vessels, heart, eyes, or kidney, or at the nervous system level. For this reason, it is essential to have effective means for the treatment and prevention of this disease.

Among the different existing types of diabetes mellitus, type 2 diabetes mellitus (DM2) accounts for between 90-95% of the total of diagnosed cases of diabetes. The development of type 2 diabetes mellitus is closely related to diet, stress and sedentary lifestyle and it is estimated that approximately 75% of the risk of developing DM2 is due to obesity. That is why the rising prevalence of obesity is accompanied by a parallel increase in the prevalence of DM2, and it is very common to find patients that must control both their weight and their glucose levels.

There are several pharmacological alternatives for controlling diabetes, for example, metformin which belongs to the biguanidine group; sulfonylureas such as glyburidie and glipizide; meglitinides such as repaglinide and nateglinide; thiazolidinediones as pioglitazone and rosiglitazone, or $\alpha$-glucosidase inhibitors such as acarbose and miglitol.

Most of these treatments are accompanied by important side effects, with the aggravating circumstance that, in addition, its effectiveness decreases after a few years of use. For this reason other non pharmacological alternative treatments have also been explored, as vitamin or mineral supplements and herbal therapies, which may be effective in the control of diabetes without the risk of side effects associated to pharmacological treatments.

Among the alternative therapies disclosed in prior art for the control of diabetes mellitus are, for example, some elements such as chromium, magnesium, potassium, selenium, vanadium or zinc, vitamins such as niacin, biotin, vitamin B6, vitamin B12, vitamin C, vitamin D, vitamin E, thiamine, and several nutrients as $\alpha$-lipoic acid, L-carnitine, N-acetylcysteine, or omega 3 fatty acids. Among them, chromium is one of the most widely used products, and therefore several clinical trials are disclosed in prior art where trivalent chromium (Cr III) is administered to patients with DM2 in order to control their glucose level, using doses usually comprised between 150 $\mu$g and 1000 $\mu$g per day, usually with notable positive effects, although some of the studies did not show efficacy, for example, the one disclosed in the article Kleefstra et al., Chromium treatment has no effect in patients with type 2 diabetes in a Western population: a randomized, double-blind, placebo-controlled trial, Diabetes Care, 2007, 30(5), 1092-1096.

In the field of the non pharmacological therapies for the treatment of diabetes, in the prior art it is also disclosed the use of herbal or plant products such as aloe, fenugreek, cinnamon, ginseng, blackberry, onion and garlic extracts, or cocoa among many others.

Thus, for example, in the article Vuksan et al., Konjac-Mannan and American Ginseng: Emerging Alternative Therapies for Type 2 Diabetes Mellitus, J.Am.Coll.Nutrit., 2001, 20(5), 370S-380S, it is disclosed how the administration of 1 g of standardised extract of American ginseng before meals, three times daily during 8 weeks, brought to the reduction of fasting blood glucose levels.

Some treatments combining two different complements have also been tested, for example, in the article Singer et al., The Effect of Chromium Picolinate and Biotin Supplementation on Glycemic Control in Poorly Controlled Patients with Type 2 Diabetes Mellitus: A Placebo-Controlled, Double-Blinded, Randomized Trial, Diabetes Technol.& Therap., 2006, 8(6), 636-643, which discloses the combined treatment with 600 $\mu$g/day of chromium, in the form of chromium picolinate, and 2 mg/day of biotin in patients with DM2 who were taking oral antihyperglycemic drugs, achieving an improvement in the glucose control.

Also, in the article Urberg et al., Evidence for synergism between chromium and nicotinic acid in the control of glucose tolerance in elderly humans, Metabolism, 1987, 36, 896-899, it is disclosed how a combined treatment with 200 $\mu$g of chromium daily and 100 mg of nicotinic acid daily for 28 days resulted in an effective control of the blood glucose levels.

In the international patent application WO-A-99/07387 it is disclosed the daily use of between 50 $\mu$g and 1000 $\mu$g of trivalent chromium and between 25 $\mu$g and 200 mg of biotin for the treatment of DM2. The chromium is in the form of picolinate.

In the international patent application WO-A-02/09693 it is disclosed a composition for the control of plasma glucose, hyperglycaemia and diabetes that comprises conjugated linoleic acid, chromium and/or ginseng.

In the international patent application WO-A-02/11564 it is disclosed a nutritional supplement for improving the control of plasma glucose and lipids. This supplement comprises a source of low glycemic index carbohydrates such as fructose or oat bran, a source of fat, a source of sterol or stanol, and a source of chromium, a source salicylic acid, and a source of ginseng.

In the Chinese patent application CN-A-1385172 it is disclosed an encapsulated composition comprising ginseng, niacin-bound trivalent organic chromium, also called chromium polynicotinate, and root from a plant of the genus *Astragalus.*

In the international patent application WO-A-2006/110491 it is disclosed a composition comprising chromium picolinate, α-lipoic acid and a magnesium salt and which is used for the prevention and treatment of diabetes. Additionally, the composition may also include one or more components selected from the group consisting of hydroxycitric acid, cinnamon extract and American ginseng.

In the international patent application WO-A-2007/098240 it is disclosed a composition comprising chromium picolinate, konjac mannan, vitamin C and α-lipoic acid and which is used for the reduction of plasma glucose levels.

In the Chinese patent application CN-A-10169537 it is disclosed a composition for the treatment of diabetes that comprises specific amounts of ginseng extract, propolis, root from the plant *Pueraria lobata (Willd.) Ohwi,* chitosan, and chromium picolinate.

In the Chinese patent application CN-A-101390902 it is disclosed a composition comprising American ginseng extract, biotin and chromium for prenatal care and prevention of birth defects and improvement of memory. US 2008/0102137 A1 relates to a composition and method for the treatment and prevention of diseases and/ or complications associated with chronic glucose metabolism destabilization.

Therefore, there is still a need to provide an alternative composition effective for the control of hyperglycaemia in patients with type 2 diabetes mellitus, especially when it is associated with obesity.

Object of the investigation

The object of the invention is a combination of active ingredients.

It is a further object of the invention a dietetic composition containing this combination.

It is a further object of the invention a pharmaceutical composition comprising said combination.

It is a further object of the invention a single-dose sachet comprising said composition.

It is a further object of the invention the use of this combination for preparing a dietetic composition.

It is a further object of the invention the use of the combination for preparing a medicine for the treatment of type 2 diabetes mellitus.

It is a further object of the invention the use of the dietetic composition to promote weight reduction.

It is a further object of the invention the use of the dietetic composition for preparing a medicine for the treatment of type 2 diabetes mellitus.

**Detailed description of the invention**

The authors of this invention have developed a combination of active ingredients that show a synergistic effect in the reduction of hyperglycaemia, and that is suitable for the treatment of type 2 diabetes mellitus. In particular, this combination can be used for the preparation of a dietetic composition that is suitable for the treatment of type 2 diabetes mellitus and to promote weight reduction in patients who also suffer from obesity.

The object of the present invention is a combination of active ingredients comprising:

    a) a trivalent chromium compound,
    b) biotin, and
    c) American ginseng root.

Trivalent chromium compounds

Chromium (Cr) is a chemical element that, in its trivalent form, Cr (III), constitutes an essential nutrient of the human diet and takes part in the metabolism of carbohydrates and lipids. Within the context of this invention, chromium compounds encompass any pharmaceutically acceptable organic or inorganic salt of Cr (III). These salts are commercially available, or alternatively can be prepared according to procedures that are known for the skilled in the art. The term "salt of chromium (III)" includes both its anhydrous form and any hydrated form.

Preferably, the trivalent chromium compounds are selected from the group consisting of chromium (III) chloride, chromium (III) chloride hexahydrate, chromium (III) picolinate, chromium (III) nicotinate, chromium (III) acetate, chromium (III) aspartate, or combinations thereof. More preferably, the trivalent chromium compound is chromium (III) chloride ($CrCl_3$), chromium (III) chloride hexahydrate, $CrCl_3 \cdot 6H_2O$, or mixtures thereof. They are both commercially available from suppliers, such as Sigma-Aldrich, for example. Within the context of the invention, the expression "equivalent trivalent chromium" is used, which refers to the content of trivalent chromium contained in a trivalent chromium compound. Thus, for example, 158.5 g of chromium (III) chloride contain 52 g of equivalent trivalent chromium, and 266.5 g of trivalent chromium chloride hexahydrate contain 52 g of equivalent trivalent chromium.

Biotin

Biotin is the common name used to designate hexahydro-2-oxo-1H-thieno-[3,4-d]imidazol-4-pentanoic acid, which is also known as vitamin B8 or vitamin H. It is a vitamin of the B group and it is an essential cofactor related to metabolism.

Biotin can be synthesized, for example, from fumaric acid as disclosed in the United Sates patent US-A-2489235. In addition, it is also commercially available from different suppliers such as, for example, Sigma-Aldrich.

American Ginseng

American ginseng (*Panax quinquefolium*) is a perennial plant of the Araliaceae family, native to eastern North America, whose root has been used for thousands of years in traditional medicine, having a revitalizing effect.

In this invention, the term ginseng refers to American ginseng.

American ginseng used in the combination of this invention can be either cultivated or wild, preferably American ginseng from cultivation is used.

The root of American ginseng in powdery from is used. This root can be milled using procedures that are known to the skilled in the art and it is commercially available, for example, from the company Kaiser Farms (Wisconsin, USA).

The authors of this invention have developed a combination comprising trivalent chromium compounds, biotin and ginseng that, surprisingly, shows a synergistic effect in the reduction of glycaemia levels, especially glycosylated haemoglobin level, in patients with DM2.

In a preferred embodiment of the invention, the combination comprises a trivalent chromium compound, biotin and American ginseng in a proportion corresponding to a weight ratio between equivalent trivalent chromium:biotin:ginseng of approximately 1:100:200000.

The term "approximately" used in this ratio means that it includes a variation margin of 20% above or below each value of said ratio, preferably a variation of 10%, and more preferably of 5%.

That means that in the previous ratio the weight of equivalent trivalent chromium is comprised between 0.8 and 1.2, preferably between 0.9 and 1.1, and still more preferably between 0.95 and 1.05. The weight of biotin is comprised between 80 and 120, preferably between 90 and 110, and still more preferably between 95 and 105. The weight of ginseng is comprised between 160000 and 240000, preferably between 180000 and 220000, and more preferably between 190000 and 210000.

Another way to express the proportion between the three components is that approximately for one part of trivalent chromium there are 100 parts of biotin and 200000 parts of ginseng, preferably for each between 0.8 and 1.2 parts of trivalent chromium, preferably between 0.9 and 1.1, and still more preferably between 0.95 and 1.05, there are between 80 and 120 parts of biotin, preferably between 90 and 110, and still more preferably between 95 and 105, and between 160000 and 240000 parts of American ginseng, preferably between 180000 and 220000, and more preferably between 190000 and 210000.

In another embodiment of the invention, the combination essentially consists of a trivalent chromium compound, biotin and American ginseng, preferably in a proportion equivalent to a weight ratio between equivalent trivalent chromium, biotin and ginseng of approximately 1:100:200000, where the term "approximately" has the same meaning previously defined.

Dietetic composition

A further object of the invention is a dietetic composition comprising the combination of the invention and at least one additional alimentary ingredient.

Within the context of the invention it is understood as a dietetic composition, which can also be called nutritional or alimentary, a composition that is to be consumed orally and that contains at least one alimentary ingredient together with dietetic complements such as, for example, vitamins, minerals, or botanic products, as those included in the combination of the invention: a trivalent chromium compound, biotin, and American ginseng.

Preferably, the type of dietetic composition in which the combination of the invention can be incorporated is one used for controlling and maintaining people's weight, more preferably, the dietetic composition is indicated for weight loss in people with overweight. Thus, the hypoglycaemic therapeutic effect of the combination of the invention is advantageously added to the anti-obesity effect of the dietetic composition.

Within the framework of the present invention the terms obesity and overweight are used indistinctly, and they both refer to a body weight above of the considered ideal.

Overweight and obesity are usually quantified according to the called "Body Mass Index" or BMI, which is the ratio between the weight and the height of the subject. Usually, a BMI comprised between 18.5 and 25 is considered normal; between 25-30 it is considered to be overweight, and when the BMI is higher than 30 it is considered to be obesity.

Thus, any dietetic composition appropriate for the maintenance and control of body weight is suitable for incorporating the combination object of the invention.

In a preferred embodiment the combination of trivalent chromium, biotin and American ginseng is incorporated in dietetic compositions appropriated for the control and maintenance of the body weight based on the administration of proteins with a high biological value and the reduction of carbohydrates and lipids as, for example, the compositions disclosed in the website http://www.pronokal.com, that are part of a diet which involves the ingestion of a normal quantity of proteins, and a reduced quantity of lipids and carbohydrates, as is described in this website.

A diet that involves the ingestion of a normal quantity of proteins is called normoproteic. In general it is considered that a normoproteic diet involves the ingestion of between 0.8 and 1.5 g of proteins per kg of body weight daily, preferably between 0.8 and 1.2 g daily.

The dietetic composition of the invention can be presented in the form of single-dose sachets that are transformed into a food to be ingested by means of the dispersion of its content in water and, occasionally, heating this dispersion; or in form of preparations ready to be consumed as, for example, bars, beverages, yogurt, biscuits, or bread.

A preferred dietetic composition according to the present invention comprises the combination of a chromium (III) compound, biotin and American ginseng, and protein.

The protein to be incorporated into the composition of the invention can be any appropriate protein used in nutritional compositions including proteins of animal origin, as, for example, milk protein, meet protein or egg protein, or proteins of vegetal origin as, for example, soya protein, pea protein, wheat protein, rice protein, among others, and mixtures thereof.

Preferably mixtures of proteins or mixtures of proteins and aminoacids are used to obtain a high biological level protein. It is understood as a "high biological level protein" the one that has the capacity to provide substantially all the aminoacids necessary for the human being. A high biological level protein is, for example, egg protein, which is virtually 100% assimilated by the human body.

Among the aminoacids appropriated to be incorporated into the dietetic compositions of the invention are, for example, L-valine, L-threonine, DL-methionine, L-isoleucine, L-leucine, L-lysine, L-tryptophan and L-phenylalanine or its mixtures.

Preferably the protein content is comprised between 30% and 80% by weight on the total weight of the dry composition, preferably between 40% and 70%, and more preferably between 45% and 55% by weight.

In a preferred embodiment the dietetic composition comprises also dietary fibres.

The dietary fibre can be of any appropriate origin, including both soluble and insoluble fibres, as, for example pectin, polydextrose, wheat bran, oat bran, among others, and mixtures thereof.

Preferably the content of dietary fibre is comprised between 0.5% and 25% by weight of the total weight of the composition, preferably between 2% and 15% by weight, and more preferably between 4% and 10% by weight.

Additionally, the composition of the invention may contain other plant extracts, nutrients, such as aminoacids, vitamins and minerals, as well as other usual additives in the food industry, such as thickeners, flavourings, sweeteners, colorants, anticaking agents, emulsifiers, and stabilisers, which are well known to those skilled in the art.

The flavourings suitable to be used in the present invention include fruit flavourings such as banana, orange, peach, pineapple or raspberry; vanilla, cinnamon, cocoa, chocolate, coffee, among others.

Some sweeteners suitable to be used in the dietetic composition of the present invention are, for example, sucralose, acesulfame K, alitame, aspartame, cyclamate, lactitol, neohesperidin DC, maltitol, saccharine, stevioside, thaumatin, among others, or their combinations.

Among the anticaking substances suitable to be used in the alimentary supplements of the invention are, for example, sodium carbonate, potassium carbonate, ammonium carbonate, magnesium carbonate, silicon oxide, among others, and their mixtures.

As stabilizers can be used, for example, potassium phosphate, sodium citrate, among others.

Among the colorant additives suitable for the alimentary compositions of the invention are, for example, curcumin, lactoflavin, lactoflavin phosphate, cochineal red, beetroot red, carotenes, or paprika.

Among the thickeners suitable to be used in the dietetic compositions of this invention are, for example, gum arabic, gum tragacanth, locust bean gum, guar gum, xanthan gum, pectin, starches, agar, alginic acid, alginate, carrageenan, and its mixtures.

Among the emulsifiers suitable for the present invention is, for example, the soy lecithin.

A further object of the invention is a single-dose sachet comprising the dietetic composition of the invention in powdery form.

In the context of the invention, it is understood as powdery from the composition that is substantially in multiparticulate form, i.e., as a powder or granulate.

The content of the single-dose sachet is used for preparing food, generally by dispersing the dietetic composition in water at room temperature and, occasionally, heating the dispersion.

In a preferred embodiment, the dietetic compositions which form part of the invention are presented in the form of single-dose sachets that contain between 10 g and 35 g of composition, i.e., contain between 3 g and 28 g of protein; preferably the sachets contain between 20 g and 30 g of composition, that corresponds to between 6 g and 24 g of protein; and more preferably between 21 g and 26 g of composition, that corresponds to between 6.3 g and 20.8 g of protein per single-dose sachet.

In a more preferred embodiment the single-dose sachets contain between 21 g and 26 g of dietetic composition, which correspond to between 10 g y 15 g of protein.

Each single-dose sachet contains also the composition of the invention, so that each one of them includes between 0.5 $\mu$g and 12 $\mu$g of trivalent chromium, preferably between 1 $\mu$g and 10 $\mu$g, and more preferably between 2 $\mu$g and 5 $\mu$g; between 0.1 mg and 2 mg of biotin, preferably between 0.2 mg and 1 mg and more preferably between 0.3 mg and 0.5 mg; and between 0.2 g and 2 g of American ginseng, preferably between 0.5 g and 1.5 g, and more preferably between 0.7 g and 0.9 g.

It is particularly preferred a single-dose sachet comprising 21 g of dietetic composition, including between 10 g and 15 g of protein, and the synergistic combination of the invention including 20 $\mu$g of trivalent chromium chloride hexahydrate, equivalent to 3.9 $\mu$g of trivalent chromium, 0.4 mg of biotin and 800 mg of American ginseng.

It is also preferred a single-dose sachet comprising 26 g of dietetic composition, including between 10 g and 15 g of protein, and the synergistic combination of the invention including 20 $\mu$g of trivalent chromium chloride hexahydrate, equivalent to 3.9 $\mu$g of trivalent chromium, 0.4 mg of biotin and 800 mg of American ginseng.

Typically, the single-dose sachets comprising the dietetic composition of the invention are administered in a quantity comprised between 1 and 6 daily, preferably between 2 and 5 sachets are administered daily.

Generally the ingestion of this composition is accompanied by the ingestion of vegetables, as for example chards, celery, watercress, borage, broccoli, zucchini, mushrooms, cauliflower, endives, spinach, lettuce, cucumbers, or green peppers; mineral complements as sodium chloride, potassium bicarbonate, magnesium oxide and calcium carbonate in order to get a balanced sodium, potassium, magnesium and calcium supply; and vitamins.

The single-dose sachets can be composed of an external paper layer, an intermediate aluminium layer, and an internal layer of polyethylene or modified polyethylenes. These sachets can be prepared by procedures available to the skilled in the art.

The dietetic composition of the invention can also be presented in the form of ready to be consumed preparations, for example, bars having a weight usually comprised between 30 g and 50 g, beverages having a volume generally comprised between 200 ml and 300 ml, yogurt having a weight comprised between 100 g and 150 g, or bread having a weight comprised between 25 g and 50 g per portion. The protein content in these ready to be consumed preparations is comprised between 10 g and 15 g.

It is a further object of invention the use of the combination of a trivalent chromium compound, biotin and American ginseng for the preparation of a dietetic composition.

The dietetic compositions of the invention in a powdery form can be prepared by following conventional procedures of the alimentary industry, which are known to the skilled in the art, as for example by sieving and mixing the solid components to obtain a homogeneous mixture. In case of incorporating liquid components, they are dosed over the other solid components so at the end a solid mixture is obtained. The ready to be consumed preparations as bars, beverages, yogurt or bread are prepared using procedures known to those skilled in the art.

Pharmaceutical composition

It is a further object of the invention a pharmaceutical composition comprising the combination of a trivalent chromium compound, biotin and American ginseng and at least one additional pharmaceutically acceptable excipient.

This pharmaceutical composition is used for the reduction of hyperglycaemia and is appropriate for the treatment of type 2 diabetes mellitus.

The combined administration of this pharmaceutical composition with a dietetic composition appropriate for the control and maintenance of body weight based on the administration of high biological value proteins, and the reduction of carbohydrates and lipids as those previously described is useful for the treatment of type 2 diabetes mellitus and promoting weight reduction in patients also suffering from obesity.

The pharmaceutical composition can be a powdery or granulated form for oral suspension, tablets, hard gelatine capsules, or syrup. Preferably the pharmaceutical composition is in powdery or granulated form for oral suspension.

In a preferred embodiment, the pharmaceutical composition that is part of the invention is in the form of a single-dose sachet.

The pharmaceutical composition can be prepared using methods well known to the skilled in the art as those described in the pharmaceutical technology manuals, as the book Remington The Science and Practice of Pharmacy, 20th Edition, Lippincott, Williams & Wilkins, Philadelphia, 2000 [ISBN: 0-683-306472].

Among the pharmaceutically acceptable excipients suitable to be included in the pharmaceutical composition are anticaking agents such as anhydrous colloidal silica, tribasic calcium phosphate, magnesium trisilicate, talc; diluents as lactose, calcium phosphate, calcium sulphate, calcium carboxymethylcellulose, microcrystalline or powdered cellulose, cellulose acetate, dextrates, dextrins, dextrose, fructose, glyceryl palmitostearate, kaolin, lactitol, magnesium carbonate, magnesium oxide, maltitol, maltodextrins, maltose, polymethacrylates, pregelatinized starch, sodium chloride, starch, sucrose, saccharose; lubricants such as magnesium stearate, calcium stearate, glyceryl palmitostearate, magnesium oxide, sodium benzoate, sodium lauryl sulphate, sodium stearyl fumarate, stearic acid, talc, glyceryl behenate; suspending agents such as xanthan gum, guar gum, alginic acid, bentonite, carbomers, sodium or calcium carboxymethylcellulose, hydroxyethylcellulose, hydroxypropylcellulose, hydroxypropylmethylcellulose, hydroxypropyl alginate, microcrystalline or powdered cellulose, anhydrous colloidal silica, dextrins, gelatins, kaolin, magnesium aluminum silicate, maltitol, povidone, sorbitan esters, tragacanth; binding agents such as magnesium trisilicate, cellulose, starch, talc, polyvinylpirrolidone, tribasic calcium phosphate; dispersing agents such as low substitution grade hydroxypropylcellulose, poloxamers, or sorbitan esters; sweeteners such as aspartame, mannitol, sorbitol, sodium saccharine, sodium cyclamate, saccharose, dextrose, fructose, glucose, inulin, isomaltose, lactitol, maltose, maltol, mannitol, sucralose, trehalose, xylitol, thaumatin; flavouring agents and/or mixtures thereof.

The physicochemical characteristics of the excipients, as well as the name of the commercial products under which they are commercialised can be found in the book R.C.Rowe et al., Handbook of Pharmaceutical Excipients, 4th edition, Pharmaceutical Press, London, 2003 [ISBN: 0-85369-472-9].

The pharmaceutical composition comprises as active ingredients between 0.5 $\mu$g and 12 $\mu$g of trivalent chromium, more preferably between 1 $\mu$g and 10 $\mu$g, and more preferably between 2 $\mu$g and 5 $\mu$g; between 0.1 mg and 2 mg of biotin, more preferably between 0.2 mg and 1 mg and more preferably between 0.3 mg and 0.5 mg; and between 0.2 g and 2 g of American ginseng, more preferably between 0.5 g and 1.5 g, and still more preferably between 0.7 g and 0.9 g.

In a preferred embodiment the pharmaceutical composition contains as active ingredients 20 $\mu$g of chromium chloride hexahydrate, equivalent to 3.9 $\mu$g of trivalent chromium, 0.4 mg of biotin, and 800 mg of American ginseng.

Efficacy assay

The methods most frequently used hitherto in the diagnosis and monitoring of diabetes are, on the one hand, the determination of Fasting Plasma Glucose, abbreviated as FPG, and on the other the Oral Glucose Tolerance Test, known by its acronym OGTT. However, there are other indirect hyperglycaemia indicators, as for example, the percentage of glycosylated haemoglobin A1C (HbA1C), which in the last years is being considered as a better indicator of the diabetes affectation, as it is concluded for example in the article International Expert Committee Report on the Role of the A1C Assay in the Diagnosis of Diabetes., Diabetes Care, 2009, 32 (7), 1327-1334. Values of HbA1c levels higher than 7% are considered as indicatives of DM2.

In the present invention the HbA1C marker is used to assess the efficacy of the combination object of the present invention for the treatment of diabetes.

The efficacy of the combination of the invention for the treatment of DM2 associated with obesity was determined in an *in vivo* assay with people diagnosed with DM2, who were non insulin dependent, and all of them with a Body Mass Index (BMI) over 30, and to whom different dietetic compositions were administered during one month, as follows:

a) Patient 1: Diet with the composition of Preparative Example 1, which does not include the combination of the invention.

b) Patient 2: Diet with the composition of Example 1, which includes the combination of the invention with trivalent chromium, biotin and American ginseng.

c) Patient 3: Diet with the composition of Preparative Example 2, which includes a combination of trivalent chromium and biotin.

d) Patient 4: Diet with the composition of Preparative Example 3, which includes American ginseng.

In Table I the results of the assay are shown, with respect to the active ingredients included in the composition:

TABLE I

| Composition | Patient | % HbA1C decrease |
|---|---|---|
| With no active ingredients | 1 | 4 |
| American ginseng | 4 | 7 |
| Trivalent chromium + biotin | 3 | 17 |
| Trivalent chromium + biotin + American ginseng | 2 | <u>39</u> |

Surprisingly a considerable synergistic effect was observed with the combination of trivalent chromium, biotin and American ginseng with a reduction of 39% of the percentage of HbA1C, compared to the 17% reduction that was obtained when the combination was formed by trivalent chromium and biotin, and compared to the 7% reduction corresponding to a composition that only included American ginseng, and against the 4% that was obtained by administering a dietetic composition that did not contain any of the active ingredients of the combination of the invention.

The reduction of the glycosylated haemoglobin (HbA1C) level achieved in Patient 2 is higher than the value expected from a simple additive effect of the effects of trivalent chromium, biotin and American ginseng, according to the data obtained for patients 3 and 4, respectively.

Therefore, it is a further object of the invention the use of the combination for the preparation of a medicament for the treatment of type 2 diabetes mellitus, i.e., it is an object of the invention the combination of a trivalent chromium compound, biotin and American ginseng for use in the treatment of type 2 diabetes mellitus.

Additionally, in all cases a considerable weight loss was also observed.

It is a further object of the invention the use of the dietetic composition to promote weight loss.

A further object of the invention is the use of the dietetic composition for the preparation of a medicament for the treatment of type 2 diabetes mellitus, i.e., it is an object of the invention the dietetic composition comprising the combination of a trivalent chromium compound, biotin and American ginseng for use in treatment of type 2 diabetes mellitus.

The following examples are meant to illustrate the invention, but not to limit it.

**Examples**

Preparative Example 1: Preparation of a dietetic composition

In a mixer provided with an stirrer for mixing solids, 73 kg of milk proteins, 19 kg of cocoa powder, 5 kg of thickening agents (xanthan gum, guar gum and sodium alginate), and 3 kg of flavouring agents and sweeteners (sucralose, and acesulfame potassium) were added, and it was mixed until a homogenous mixture was obtained.

All the ingredients were sieved through a 3 mm sieve before incorporating them into the mixer.

The powdery mixture obtained was packed into individual sachets, containing 25 g per sachet.

The dispersion of the sachet content in water at room temperature, followed by heating of the dispersion obtained, provides a hot chocolate beverage with high protein content.

Similarly, dietetic compositions of several flavours can be obtained, for example, coconut, strawberry, grapefruit, tomato soup, vegetable cream, omelette basis, among others.

Preparative Example 2: Preparation of a dietetic composition containing a combination of chromium (III) chloride and biotin.

It was prepared similarly as Preparative Example 1, incorporating to the mixture 1.6 g of biotin and 80 mg of trivalent chromium chloride hexahydrate.

The mixture obtained was packed into individual sachets, which contained 25 g per sachet.

Thus, each sachet contained 0.4 mg of biotin and 20 $\mu$g of trivalent chromium chloride hexahydrate, equivalent to 3.9 $\mu$g of trivalent chromium.

Preparative Example 3: Preparation of a dietetic composition containing American ginseng

It was prepared according to the same procedure followed in Preparative Example 1, additionally adding to the mixture 3.2 kg of American ginseng.

The resulting mixture was packed into individual sachets, which contained 25.8 g per sachet.

Thus, each sachet contained 800 mg of American ginseng.

Example 1: Preparation of a composition containing a combination of chromium chloride (III), biotin and American ginseng

It was prepared, according to an analogous procedure as used in Preparative Example 1, but additionally incorporating to the mixture, 3.2 kg of American ginseng, 1.65 g of biotin and 82 mg of trivalent chromium chloride hexahydrate.

The mixture obtained was packed into individual sachets, which contained 25.8 g per sachet.

Thus, each sachet contained: 0.4 mg of biotin, 20 $\mu$g of trivalent chromium chloride hexahydrate, equivalent to 3.9 $\mu$g of trivalent chromium and 800 mg of American ginseng.

Example 2: Efficacy study

For performing the efficacy study of the combination of the invention 4 patients were recruited aged between 30 and 65 years, diagnosed with type 2 diabetes mellitus of less than 10 years of evolution, non insulin-dependents, and all of them with a Body Mass Index (BMI) higher than 30. The duration of the study was one month, during which the patients followed a diet based on the dietetic compositions of Example 1 and the Preparative Examples 1, 2 and 3, consisting in taking 5 sachets daily, occasionally complemented with vegetables, minerals and vitamins according to the following pattern:

- Breakfast: 1 sachet with the dietetic composition of the corresponding example complemented with 1 g of sodium chloride; 0.6 g of potassium bicarbonate; 0.3 g of magnesium oxide; and a capsule with vitamins and minerals with the following composition: 800 $\mu$g of vitamin A, 60 mg of vitamin C, 5 $\mu$g of vitamin D3, 10 mg of vitamin E, 1.4 mg of vitamin B1, 1.6 mg of vitamin B2, 18 mg of niacin, 2 mg of vitamin B6, 200 $\mu$g of folic acid, 1 $\mu$g of vitamin B12, 6 mg of pantothenic acid, 14 mg of iron, 50 $\mu$g of selenium, 150 $\mu$g of iodine, 15 mg of zinc, 3.5 mg of magnesium, and 150 $\mu$g of molybdenum.
- Mid-morning: 1 sachet with the dietetic composition of the corresponding example complemented with 0.625 g of calcium carbonate.
- Lunch: 1 sachet with the dietetic composition of the corresponding example complemented with free consumption of vegetables with low carbohydrate content (for example, chard, celery, watercress, borage, broccoli, zucchini, mushrooms, cauliflower, endives, spinach, lettuce, cucumber, or green peppers), 1 g of sodium chloride; and 1.2 g of potassium bicarbonate.
- Afternoon: 1 sachet with the dietetic composition of the corresponding example, and 0.625 g of calcium carbonate.
- Dinner: 1 sachet with the dietetic composition of the corresponding example complemented with free consumption of vegetables with low carbohydrate content (for example, chard, celery, watercress, borage, broccoli, zucchini, mushrooms, cauliflower, endives, spinach, lettuce, cucumber, or green peppers), 1 g of sodium chloride; and 1.2 g of potassium bicarbonate, and 0.3 g of magnesium oxide.
- Snack: 1 sachet with the dietetic composition of the corresponding example.

Patients consumed al least 2 litres of water per day, preferably still, and also it could be in the form of 100% natural coffee, unsweetened infusions, white tea or black tea.

Furthermore, patients consumed 1 tablespoon daily of extra virgin olive oil.

A control was performed measuring weight, BMI, visceral fat and glycosylated haemoglobin (HbA1C), before starting the study (Start) and at its conclusion (End). To compare the efficacy in the control of the hyperglycaemia of the four treatments, the % of HbA1C reduction index was used that was calculated for each patient according to the following formula:

$$\frac{HbA1C_{Start} - HbA1C_{End}}{HbA1C_{Start}} \times 100$$

The results obtained are shown in Table II:

TABLE II

| Patient | 1 | | 3 | | 4 | | 2 | |
|---|---|---|---|---|---|---|---|---|
| Composition | Preparative Example 1 | | Preparative Example 2 | | Preparative Example 3 | | Example 1 | |
| | *Start* | *End* | *Start* | *End* | *Start* | *End* | *Start* | *End* |
| Weight (kg) | 79.5 | 71.5 | 98.3 | 90.2 | 83.1 | 74.3 | 124.1 | 109.7 |
| BMI | 30.3 | 27.2 | 31.4 | 28.8 | 32.1 | 28.7 | 37.1 | 32.8 |
| Visceral fat (kg) | 13 | 10 | 16 | 13 | 12 | 9 | 17 | 14 |
| HbA1C(%) | 7.3 | 7.0 | 7.2 | 6.0 | 7.2 | 6.7 | 10.7 | 6.5 |
| % HbA1C reduction | | 4 | | 17 | | 7 | | <u>39</u> |

Patients have been subjected to the following diets:

a) Patient 1: Diet with the composition of Preparative Example 1, which does not include the combination of the invention.
b) Patient 2: Diet with the composition of Example 1, which includes the combination of the invention.
c) Patient 3: Diet with the composition of Preparative Example 2, which includes a combination of trivalent chromium and biotin.
d) Patient 4: Diet with the composition of Preparative Example 3, which includes American ginseng.

In Table III the results obtained are shown with respect to the active ingredients included in the composition:

TABLE III

| Composition | % HbA1C reduction | Weight decrease (kg) |
|---|---|---|
| Without active ingredients | 4 | 8.0 |
| American ginseng | 7 | 8.8 |
| Trivalent chromium + biotin | 17 | 8.1 |
| Trivalent chromium + biotin + American ginseng | <u>39</u> | 14.4 |

A considerable synergistic effect was observed with the combination of trivalent chromium, biotin and American ginseng (Patient 2, treated with the complement of Example 1), according to the 39% rate of HbA1C reduction versus the 17% of Patient 3, who took the combination of trivalent chromium and biotin, and versus the 7% reduction of Patient 4, who took the dietetic complement enhanced only with ginseng. The glycosiylated haemoglobin (HbA1C) reduction rate achieved in Patient 2 is higher than the value that would have been expected in case of having a simple additive effect of the effects of the trivalent chromium, biotin and American ginseng, according to the data obtained for patients 3 and 4, respectively.

Weigh reduction was also observed in all patients, caused by the use of the dietetic composition of the invention designed to achieve a weight reduction. The absolute weight decrease is higher in patient 2, since his initial weight was considerably higher than in the other patients.

**Claims**

1.   Combination of active ingredients, **characterized in that** it comprises:

a) a trivalent chromium compound,
b) biotin, and
c) American ginseng root.

2.   Combination according to claim 1, **characterized in that** the ratio expressed in weight between equivalent trivalent chromium, biotin and American ginseng is approximately 1:100:200000.

3. Combination according to claims 1 or 2, **characterized in that** the trivalent chromium compound is selected from the group consisting of: chromium (III) chloride, chromium (III) chloride hexahydrate, chromium (III) picolinate, chromium (III) nicotinate, chromium (III) acetate, chromium (III) aspartate, or combinations thereof.

4. Dietetic composition comprising the combination according to any of claims 1 to 3, and at least one additional alimentary ingredient.

5. Dietetic composition according to claim 4, **characterized in that** it comprises protein.

6. Dietetic composition according to claim 5, **characterized in that** the protein is selected from the group consisting of milk protein, meet protein, egg protein, soya protein, pea protein, wheat protein, rice protein, and mixtures thereof.

7. Dietetic composition according to any of claims 4 to 6, **characterized in that** it comprises dietary fibre.

8. Single-dose sachet **characterized in that** it comprises the composition of any of claims 4 to 7 in powdery form.

9. Single-dose sachet according to claim 8, **characterized in that** it contains between 10 g and 35 g of the composition of any of claims 4 to 7.

10. Single-dose sachet according to claims 8 or 9, **characterized in that** it comprises between 3 g and 28 g of protein.

11. Single-dose sachet according to any of claims 8 to 10, **characterized in that** it comprises 26 g of the dietetic composition of any of claims 4 to 7, and comprising 20 $\mu$g of trivalent chromium chloride hexahydrate, 0.4 mg of biotin and 800 mg of American ginseng root.

12. Pharmaceutical composition **characterized in that** it comprises the combination of any of claims 1 to 3 and at least one additional pharmaceutically acceptable excipient.

13. Combination according to any of claims 1 to 3 for use in the treatment of type 2 diabetes mellitus.

14. Dietetic composition according to any of claims 4 to 7 for use in the treatment of type 2 diabetes mellitus.

15. Dietetic composition according to any of claims 4 to 7 for use in promoting weight loss.

**Patentansprüche**

1. Wirkstoffkombination, **dadurch gekennzeichnet, dass** sie Folgendes umfasst:

   a) eine dreiwertige Chromverbindung,
   b) Biotin und
   c) Amerikanische Ginsengwurzel.

2. Kombination nach Anspruch 1, **dadurch gekennzeichnet, dass** das Gewichtsverhältnis zwischen äquivalentem dreiwertigem Chrom, Biotin und Amerikanischer Ginsengwurzel etwa 1:100:200.000 beträgt.

3. Kombination nach Anspruch 1 oder 2, **dadurch gekennzeichnet, dass** die dreiwertige Chromverbindung ausgewählt ist aus der Gruppe bestehend aus: Chrom(III)-chlorid, Chrom(III)-chloridhexahydrat, Chrom(III)-picolinat, Chrom(III)-nicotinat, Chrom(III)-acetat, Chrom(III)-aspartat oder Kombinationen davon.

4. Diätetische Zusammensetzung, die die Kombination nach einem der Ansprüche 1 bis 3 und mindestens einen zusätzlichen Ernährungsinhaltsstoff umfasst.

5. Diätetische Zusammensetzung nach Anspruch 4, **dadurch gekennzeichnet, dass** sie Protein umfasst.

6. Diätetische Zusammensetzung nach Anspruch 5, **dadurch gekennzeichnet, dass** das Protein ausgewählt ist aus der Gruppe bestehend aus Milchprotein, Fleischprotein, Eiprotein, Sojaprotein, Erbsenprotein, Weizenprotein, Reisprotein und Mischungen davon.

**7.** Diätetische Zusammensetzung nach einem der Ansprüche 4 bis 6, **dadurch gekennzeichnet, dass** sie Ballaststoffe umfasst.

**8.** Einzelportionspackung, **dadurch gekennzeichnet, dass** sie die Zusammensetzung aus einem der Ansprüche 4 bis 7 in Pulverform umfasst.

**9.** Einzelportionspackung nach Anspruch 8, **dadurch gekennzeichnet, dass** sie zwischen 10 g und 35 g der Zusammensetzung aus einem der Ansprüche 4 bis 7 enthält.

**10.** Einzelportionspackung nach Anspruch 8 oder 9, **dadurch gekennzeichnet, dass** sie zwischen 3g und 28 g Protein umfasst.

**11.** Einzelportionspackung nach einem der Ansprüche 8 bis 10, **dadurch gekennzeichnet, dass** sie 26 g der diätetischen Zusammensetzung aus einem der Ansprüche 4 bis 7 und 20 μg dreiwertiges Chromchloridhexahydrat, 0,4 mg Biotin und 800 mg Amerikanische Ginsengwurzel umfasst.

**12.** Pharmazeutische Zusammensetzung, **dadurch gekennzeichnet, dass** sie die Kombination aus einem der Ansprüche 1 bis 3 und mindestens einen zusätzlichen pharmazeutisch unbedenklichen Trägerstoff umfasst.

**13.** Kombination nach einem der Ansprüche 1 bis 3 zur Verwendung bei der Behandlung von Typ 2-Diabetes mellitus.

**14.** Diätetische Zusammensetzung nach einem der Ansprüche 4 bis 7 zur Verwendung bei der Behandlung von Typ 2-Diabetes mellitus.

**15.** Diätetische Zusammensetzung nach einem der Ansprüche 4 bis 7 zur Verwendung beim Fördern der Gewichtsabnahme.

**Revendications**

**1.** Combinaison de principes actifs, **caractérisée en ce qu'**elle comprend :

a) un composé de chrome trivalent,
b) de la biotine, et
c) de la racine de ginseng américain.

**2.** Combinaison selon la revendication 1, **caractérisée en ce que** le rapport exprimé en poids entre l'équivalent de chrome trivalent, la biotine et le ginseng américain est approximativement de 1/100/200000.

**3.** Combinaison selon les revendications 1 ou 2, **caractérisée en ce que** le composé de chrome trivalent est choisi dans le groupe constitué de : chlorure de chrome (III), chlorure de chrome (III) hexahydraté, picolinate de chrome (III), nicotinate de chrome (III), acétate de chrome (III), aspartate de chrome (III), ou leurs combinaisons.

**4.** Composition diététique comprenant la combinaison selon l'une quelconque des revendications 1 à 3, et au moins un composant alimentaire supplémentaire.

**5.** Composition diététique selon la revendication 4, **caractérisée en ce qu'**elle comprend une protéine.

**6.** Composition diététique selon la revendication 5, **caractérisée en ce que** la protéine est choisie dans le groupe constitué de protéine du lait, protéine de viande, protéine d'oeuf, protéine de soja, protéine de pois, protéine de blé, protéine de riz, et leurs mélanges.

**7.** Composition diététique selon l'une quelconque des revendications 4 à 6, **caractérisée en ce qu'**elle comprend une fibre alimentaire.

**8.** Sachet monodose **caractérisé en ce qu'**il comprend la composition selon l'une quelconque des revendications 4 à 7 sous forme de poudre.

9. Sachet monodose selon la revendication 8, **caractérisée en ce qu'**il contient entre 10 g et 35 g de la composition selon l'une quelconque des revendications 4 à 7.

10. Sachet monodose selon les revendications 8 ou 9, **caractérisé en ce qu'**il comprend entre 3 g et 28 g de protéine.

11. Sachet monodose selon l'une quelconque des revendications 8 à 10, **caractérisé en ce qu'**il comprend 26 g de la composition diététique selon l'une quelconque des revendications 4 à 7, et comprenant 20 µg de chlorure de chrome trivalent hexahydraté, 0,4 mg de biotine et 800 mg de racine de ginseng américain.

12. Composition pharmaceutique **caractérisée en ce qu'**elle comprend la combinaison selon l'une quelconque des revendications 1 à 3 et au moins un excipient pharmaceutiquement acceptable supplémentaire.

13. Combinaison selon l'une quelconque des revendications 1 à 3 pour une utilisation dans le traitement du diabète de type 2.

14. Composition diététique selon l'une quelconque des revendications 4 à 7 pour une utilisation dans le traitement du diabète de type 2.

15. Composition diététique selon l'une quelconque des revendications 4 à 7 pour une utilisation dans l'activation de la perte de poids.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9907387 A
- WO 0209693 A
- WO 0211564 A
- CN 1385172 A
- WO 2006110491 A

- WO 2007098240 A
- CN 10169537 A
- CN 101390902 A
- US 20080102137 A1
- US 2489235 A


**Non-patent literature cited in the description**

- **SHAW et al.** Global estimates of the prevalence of diabetes for 2010 and 2030. *Diabetes Res.Clin.Pract.,* 2010, vol. 87, 4-14
- **KLEEFSTRA et al.** Chromium treatment has no effect in patients with type 2 diabetes in a Western population: a randomized, double-blind, placebo-controlled trial,. *Diabetes Care,* 2007, vol. 30 (5), 1092-1096
- **VUKSAN et al.** Konjac-Mannan and American Ginseng: Emerging Alternative Therapies for Type 2 Diabetes Mellitus. *J.Am.Coll.Nutrit.,* 2001, vol. 20 (5), 370S-380S
- **SINGER et al.** The Effect of Chromium Picolinate and Biotin Supplementation on Glycemic Control in Poorly Controlled Patients with Type 2 Diabetes Mellitus: A Placebo-Controlled, Double-Blinded, Randomized Trial. *Diabetes Technol.& Therap.,* 2006, vol. 8 (6), 636-643

- **URBERG et al.** Evidence for synergism between chromium and nicotinic acid in the control of glucose tolerance in elderly humans. *Metabolism,* 1987, vol. 36, 896-899
- pharmaceutical technology manuals. Remington The Science and Practice of Pharmacy. Lippincott, Williams & Wilkins, 2000
- **R.C.ROWE et al.** Handbook of Pharmaceutical Excipients. Pharmaceutical Press, 2003
- International Expert Committee Report on the Role of the A1C Assay in the Diagnosis of Diabetes. *Diabetes Care,* 2009, vol. 32 (7), 1327-1334